# EUROPÄISCHE PATENTANMELDUNG

(11) **EP 2 062 566 A1**
(43) Veröffentlichungstag der Anmeldung: **27.05.2009**
(21) Anmeldenummer: 08164754.7
(22) Anmeldetag: 22.09.2008
(51) Int. Cl.: A61K 8/37, A61K 8/49, A61Q 17/04

(54) **Kosmetische und dermatologische Formulierungen enthaltend Isononylbenzoat**

(30) Priorität: 21.11.2007 DE 102007055483
(71) Anmelder: Evonik Goldschmidt GmbH, 45127 Essen (DE)
(72) Erfinder: Springer, Dr. Oliver, 46485, Wesel (DE); Thum, Dr. Oliver, 40880, Ratingen (DE); Meyer, Dr. Jürgen, 45134, Essen (DE); Jenni, Dr. Klaus, 45357, Essen (DE)

(57) **Zusammenfassung**

Gegenstand der Erfindung ist die Verwendung von Isononylbenzoat zur Herstellung einer kosmetischen, dermatologischen oder pharmazeutischen Formulierung.

## Beschreibung

### Gebiet der Erfindung

Die vorliegende Erfindung betrifft die Verwendung von Isononylbenzoat zur Herstellung einer kosmetischen, dermatologischen oder pharmazeutischen Formulierung. Das Isononylbenzoat zeichnet sich sowohl dadurch aus, dass es gute sensorische Eigenschaften verleiht, als auch durch sehr gute Lösungseigenschaften für aktive Substanzen, insbesondere organische UV-Lichtschutzfilter.

### Stand der Technik

Im Bereich kosmetischer Emulsionen für die Haut- und Haarpflege werden vom Verbraucher eine Vielzahl von Anforderungen gestellt: Abgesehen von den reinigenden und pflegenden Effekten, die den Anwendungszweck bestimmen, wird Wert auf so unterschiedliche Parameter wie höchstmögliche dermatologische Verträglichkeit, gute rückfettende Eigenschaften, elegantes Erscheinungsbild, optimaler sensorischer Eindruck und Lagerstabilität gelegt.

Zubereitungen, die zur Reinigung und Pflege der menschlichen Haut und der Haare eingesetzt werden, enthalten in der Regel neben einer Reihe von oberflächenaktiven Substanzen, vor allem Ölkörper und Wasser. Als Ölkörper/Emollients werden beispielsweise Kohlenwasserstoffe, Esteröle sowie pflanzliche und tierische Öle/Fette/Wachse eingesetzt. Um die hohen Anforderungen des Marktes bezüglich sensorischer Eigenschaften und optimaler dermatologischer Verträglichkeit zu erfüllen, werden kontinuierlich neue Ölkörper und Emulgator-Gemische entwickelt und getestet.

Weiterhin ist bekannt, dass insbesondere der ultraviolette Anteil von natürlichen und künstlichen Lichtquellen (UV-A 320-390 nm; UV-B 280-320 nm; UV-C 100 oder 200-280 nm) in größeren Mengen zur Schädigung der menschlichen Haut führt. Die UV-A Strahlung bewirkt hauptsächlich die Hautalterung (Oberhautverdünnung und Bindegewebedegeneration, Pigmentstörungen), während UV-B und UV-C zu Sonnenbrand und Hautkrebs führen.

Das insbesondere in den letzen Jahren veränderte Freizeitverhalten mit verlängerten Aufenthalten im Freien und insbesondere extensives Sonnenbaden zur Erzielung der "gesunden Bräune" haben allerdings vor dem Hintergrund der medizinischen Erkenntnisse, des Bewusstseins der mangelnden natürlichen Schutzmechanismen der Haut durch Pigmentbildung und Sonnengewöhnung durch Verdickung der Hornschicht, die Notwendigkeit eines ausreichenden Schutzes vor intensiver UV-Strahlung weit in den Vordergrund gerückt. Deutlich verstärkt wurde sie durch die Diskussion der Abnahme und Verdünnung des antarktischen Ozonloches und der damit einhergehenden Steigerung der Intensität der UV-A und UV-B Strahlung auf der Erdoberfläche.

Das wird deutlich an den in den letzten Jahren steigenden Umsätzen von Produkten mit hohen Sonnenschutzfaktoren (sun protection factor: SPF). Dies sind in erster Linie immer noch die klassischen Sonnenschutzformulierungen (Sonnenmilch, Sonnenöl) mit dem primären Anwendungszweck Sonnenbaden, jedoch zunehmend auch die sogenannten Pflegeprodukte für Gesicht, Körper und Haar wie Tages- und Nachtcremes, Conditioner, Lotionen, (Hydro-, Lipo)Gelen, (Lippen-)Stiften und Sprays, pharmazeutische Formulierungen und in geringem Umfang Produkte der dekorativen Kosmetik, die überwiegend in Form von Ölen und flüssigen, cremeförmigen oder salben/pastenartigen W/O- und O/W- Emulsionen im Handel sind.

Der Lichtschutzfaktor LSF oder auch SPF ist ein Koeffizient, welcher das Vermögen eines Produktes ausdrückt, Sonnenbrand durch die Sonne zu verhindern. Lichtschutz mit einem Faktor von 60 schützt daher doppelt so lange wie ein Produkt mit Faktor 30 und entsprechend dreimal solange wie ein Produkt mit Faktor 20 vor dem Auftreten eines Sonnenbrandes.

Diese höheren Lichtschutzfaktoren werden in den meisten Fällen erzeugt durch eine Erhöhung der Konzentration von UV-Lichtschutzfiltern in der Formulierung.

Seit 1995 werden die Lichtschutzfaktoren nach derselben internationalen Norm (COLIPA) gemessen, die einen Vergleich zwischen den Produkten der verschiedenen Hersteller erlaubt.

Bei den häufigen und großflächigen Anwendungen ist es nicht ausgeschlossen, dass die hochdosierten Filter (ca. 3 bis 30 Gew.-% der Formulierung) in Gramm-Mengen auf die Haut aufgetragen werden. Diese Mengen an Filtersubstanzen müssen aber gelöst und homogen und stabil in der Formulierung eingearbeitet worden sein.

Zum Lösen dieser Substanzen wird häufig von ölartigen Komponenten Gebrauch gemacht, die ein gutes Lösungsvermögen für die Filtersubstanzen besitzen. So werden unter anderem auch bestimmte Esteröle eingesetzt. Eine verwendete Verbindungsklasse sind hier aliphatische Benzoesäureester. Ein typischer Vertreter dieser Verbindungsklasse ist die Verbindung Tegosoft® TN (C12-C15 Alkylbenzoat), welche besonders häufig als Lösungsmittel für UV-Lichtschutzfiltern eingesetzt wird.

Zum Schutze gegen UV-B-Strahlung sind zahlreiche Verbindungen bekannt, bei denen es sich zumeist um Derivate des 3-Benzylidencamphers, der 4-Aminobenzoesäure, der Zimtsäure, der Salicylsäure, des Benzophenons sowie auch des 2-Phenylbenzimidazols handelt.

Auch für den Bereich zwischen etwa 320 nm und etwa 400 nm, den sogenannten UV-A-Bereich, ist es wichtig, Filtersubstanzen zur Verfügung zu haben, da auch dessen Strahlen Schäden hervorrufen können. So ist erwiesen, dass UV-A-Strahlung zu einer Schädigung der elastischen und kollagenen Fasern des Bindegewebes führt, was die Haut vorzeitig altern lässt, und dass sie als Ursache zahlreicher phototoxischer und photoallergischer Reaktionen zu sehen ist. Der schädigende Einfluss der UV-B-Strahlung kann durch UV-A-Strahlung verstärkt werden.

Ein UV-B-Filter ist der 4,4',4''-(1,3,5-Triazin-2,4,6-triyltriimino)-tris-benzoesäure-tris(2-ethylhexylester), synonym: 2,4,6-Tris-[anilino(p-carbo-2'-ethyl-1'-hexyl-oxy)]- 1,3,5-triazin. Diese UV-B-Filtersubstanz wird von der BASF AG unter der Warenbezeichnung UVINUL^{™} T 150 vertrieben und zeichnet sich durch gute UV-Absorptionseigenschaften aus.

Der Hauptnachteil dieses UV-B-Filters ist die schlechte Löslichkeit in Lipiden. Bekannte Lösungsmittel für diesen UV-B-Filter können maximal ca. 15 Gew.-% dieses Filters lösen, entsprechend etwa 1-1,5 Gew.-% gelöster, und damit aktiver, UV-Lichtschutzfilter.

Das Isononylbenzoat ist der Ester der Benzoesäure mit Isononanol. Isononylbenzoat ist eine an sich bekannte Verbindung, die als schnell gelierender niedrigviskoser Weichmacher für Kunststoffe (PVC, PAMA, PVB, etc.) und Klebstoffe eingesetzt wird und u.a. von der Oxeno Olefinchemie GmbH unter dem Handelsnamen Vestinol® INB vertrieben wird. Isononanol wird u.a. unter den Chemical Abstract Service (CAS) Nummern 27458-94-2, 68515-81-1 oder auch 3452-97-9 geführt. Die Herstellung erfolgt in der Regel durch Veresterung von Isononanol mit Benzoesäure wie in DE 10217186 beschrieben. Die Veresterungs- und Behandlungsbedingungen können dabei auch variiert werden, wie beispielsweise in US 6635775 für andere Benzoesäureester beschrieben.

Aufgabe der Erfindung war es, einen Ölkörper bereitzustellen, der neben guten sensorischen Eigenschaften, wie Farbe, Geruch und Hautgefühl, auch eine gute Löslichkeit für aktive Substanzen, insbesondere für organische UV-Lichtschutzfilter, wie z.B. Triazine, so wie vorzugsweise auch für Deodorant- und Antitranspirantwirkstoffe besitzt.

Weitere nicht explizit genannte Aufgaben ergeben sich aus dem Kontext der nachfolgenden Beschreibung, der Beispiele sowie der Ansprüche.

### Beschreibung der Erfindung

Überraschenderweise wurde gefunden, dass Isononylbenzoat diese Anforderungen erfüllt.

Ein Gegenstand der Erfindung ist die Verwendung von Isononylbenzoat zur Herstellung einer kosmetischen, dermatologischen oder pharmazeutischen Formulierung.
Ein weiterer Gegenstand der Erfindung sind kosmetische, dermatologische oder pharmazeutische Formulierungen enthaltend Isononylbenzoat.

Die erfindungsgemäße Verwendung von Isononylbenzoat und die erfindungsgemäßen Isononylbenzoat enthaltenden Formulierungen, werden nachfolgend beispielhaft beschrieben, ohne dass die Erfindung auf diese beispielhaften Ausführungsformen beschränkt sein soll. Sind nachfolgend Bereiche, allgemeine Formeln oder Verbindungsklassen angegeben, so sollen diese nicht nur die entsprechenden Bereiche oder Gruppen von Verbindungen umfassen, die explizit erwähnt sind, sondern auch alle Teilbereiche und Teilgruppen von Verbindungen, die durch Herausnahme von einzelnen Werten (Bereichen) oder Verbindungen erhalten werden können. Werden im Rahmen der vorliegenden Beschreibung Dokumente zitiert, so soll deren Inhalt vollständig zum Offenbarungsgehalt der vorliegenden Erfindung gehören. Werden im Rahmen der vorliegenden Erfindung Verbindungen, wie z. B. organomodifizierte Polysiloxane, beschrieben, die verschiedene Einheiten mehrfach aufweisen können, so können diese statistisch verteilt (statistisches Oligomer) oder geordnet (Blockoligomer) in diesen Verbindungen vorkommen. Angaben zu Anzahl von Einheiten in solchen Verbindungen sind als Mittelwert, gemittelt über alle entsprechenden Verbindungen zu verstehen. Alle angegebenen Prozent (%) sind wenn nicht anders angegeben Massenprozent.

Als Isononylbenzoat wird im Folgenden mindestens eine Substanz der allgemeinen Formel (I) 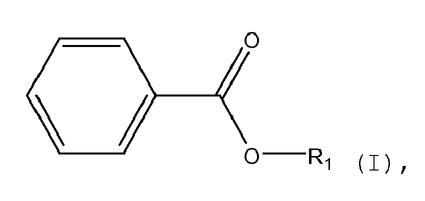 wobei R₁ ein unverzweigter oder verzweigter C₉-Alkylrest sein kann, sowie Isomerenmischungen hiervon, verstanden.

Erfindungsgemäß wird mindestens eine Substanz der allgemeinen Formel (I) mit R₁ gleich unverzweigter oder verzweigter C₉-Alkylrest zur Herstellung einer kosmetischen, dermatologischen oder pharmazeutischen Formulierung, insbesondere Sonnenschutzpräparaten, verwendet. Es können aber auch einzelne Isomere, beispielsweise der Benzoesäureester des n-Nonanols, 2-Methyloctanols, 2-Ethylheptanols, 2-Propylhexanols, 4-Methyloctanols, 2,3-Dimethylheptanols, 3-Ethylheptanols, 2-Propyl-3-methyl-penatols, 2-ethyl-4-methyl-hexanols, 2,5-Dimethylheptanols, 6-Methyloctanols, 4,5-Dimethylheptanols, 2,3,4-Trimethylhexanols, 3-Ethyl-4-methylhexanols und/oder des 3,5,5-Trimethylhexanols eingesetzt werden. Bevorzugt verwendet werden Gemische der Isomere. Besonders bevorzugt werden eine oder mehrere Substanzen der Formel (I) verwendet, mit der Maßgabe, dass die durch Verseifung der verwendeten Substanz(en) erhaltenen Nonylalkohole weniger als 10 Mol % 3,5,5-Trimethylhexanol enthalten.

Die kosmetischen, dermatologischen oder pharmazeutischen Formulierungen sowie die Pflege- und Reinigungsmittel können z. B. mindestens eine zusätzliche Komponenten enthalten, ausgewählt aus der Gruppe der
Emollients,
Emulgatoren und Tenside,
Verdicker/Viskositätsregler/Stabilisatoren,
UV-Lichtschutzfilter,
Antioxidantien,
Hydrotrope (oder Polyole),
Fest- und Füllstoffe,
Filmbildner,
Perlglanzadditive,
Deodorant- und Antitranspirantwirkstoffe,
Insektrepellentien,
Selbstbräuner,
Konservierungsstoffe,
Konditioniermittel,
Parfüme,
Farbstoffe,
Biogene Wirkstoffe,
Pflegeadditive,
Überfettungsmittel
Lösungsmittel.

Als Emollients können alle kosmetischen Öle insbesondere Mono- oder Diester von linearen und/oder verzweigten Mono- und/oder Dicarbonsäuren mit 2 bis 44 C-Atomen mit linearen und/oder verzweigten gesättigten oder ungesättigten Alkoholen mit 1 bis 22 C-Atomen eingesetzt werden. Ebenso sind die Veresterungsprodukte aliphatischer, difunktioneller Alkohole mit 2 bis 36 C-Atomen mit monofunktionellen aliphatischen Carbonsäuren mit 1 bis 22 C-Atomen einsetzbar. Des Weiteren eignen sich langkettige Arylsäureester wie z. B. Ester der Benzoesäure mit linearen und/oder verzweigten C₆-C₂₂-Alkoholen, oder auch Benzoesäureisostearylester, Benzoesäurebutyloctylester oder Benzoesäureoctyldocecylester. Weitere als Emollients und Ölkomponenten geeignete Monoester sind z.B. die Methylester und Isopropylester von Fettsäuren mit 12 bis 22 C-Atomen, wie z. B. Methyllaurat, Methylstearat, Methyloleat, Methylerucat, Isopropylmyristat, Isopropylpalmitat, Isopropylstearat, Isopropyloleat möglich. Andere geeignete Monoester sind z.B. n-Butylstearat, n-Hexyllaurat, n-Decyloleat, Isooctylstearat, Isononylpalmitat, Isononylisononanoat, 2-Ethylhexyllaurat, 2-Ethylhexylpalmitat, 2-Ethylhexylstearat, 2-Hexyldecylstearat, 2-Octyldodecylpalmitat, Oleyloleat, Oleylerucat, Erucyloleat sowie Ester, die aus technischen aliphatischen Alkoholschnitten und technischen, aliphatischen Carbonsäuregemischen erhältlich sind, z. B. Ester aus ungesättigten Fettalkoholen mit 12 bis 22 C-Atomen und gesättigten und ungesättigten Fettsäuren mit 12 bis 22 C-Atomen wie sie aus tierischen und pflanzlichen Fetten zugänglich sind. Geeignet sind aber auch natürlich vorkommende Monoester- bzw. Wachsester-Gemische wie sie z. B. im Jojobaöl oder im Spermöl vorliegen. Geeignete Dicarbonsäureester sind z. B. Di-n-butyl-adipat, Di-n-butyl-sebacat, Di-(2-ethylhexyl)-adipat, Di-(2-hexyldecyl)-succinat, Di-isotridecylazelat. Geeignete Diolester sind z.B. Ethylenglycoldioleat, Ethylenglycol-di-isotridecanoat, Propylenglycol-di-(2-ethylhexanoat), Butandiol-di-isostearat, Butandiol-di-caprylat/caprat und Neopentylglycol-di-caprylat. Weitere Fettsäureester, die als Emollients eingesetzt werden können, sind z. B. Dicaprylylcarbonat, Diethylhexylcarbonat. Ebenso als Emollients und Ölkomponente können längerkettige Triglyceride, d.h. dreifache Ester des Glycerins mit drei Säuremolekülen, wovon mindestens eine längerkettig ist, eingesetzt werden. Hier seien beispielhaft Fettsäuretriglyceride erwähnt; als solche können beispielsweise natürliche, pflanzliche Öle, z.B. Olivenöl, Sonnenblumenöl, Sojaöl, Erdnußöl, Rapsöl, Mandelöl, Sesamöl, Avocadoöl, Rizinusöl, Kakaobutter, Palmöl aber auch die flüssigen Anteile des Kokosöls oder des Palmkernöls sowie tierische Öle wie z.B. Haifischlebertran, Dorschleberöl, Walöl, Rindertalg und Butterfett, Wachse wie Bienenwachs, Karnaubapalmwachs, Spermazet, Lanolin und Klauenöl, die flüssigen Anteile des Rindertalgs oder auch synthetische Triglyceride von Capryl-Caprinsäure-Gemischen, Triglyceride aus technischer Ölsäure, Triglyceride mit Isostearinsäure, oder aus Palmitinsäure-Ölsäure-Gemischen als Emollients und Ölkomponenten eingesetzt werden. Weiterhin können Kohlenwasserstoffe, insbesondere auch flüssige Paraffine und Isoparaffine eingesetzt werden. Beispiele für einsetzbare Kohlenwasserstoffe sind Paraffinöl, Isohexadecan, Polydecen, Vaseline, Paraffinum perliquidum, Squalan, Ceresin. Weiterhin sind auch lineare oder verzweigte Fettalkohole wie Oleylalkohol oder Octyldodecanol, sowie Fettalkoholether wie Dicaprylyl Ether einsetzbar. Geeignete Siliconöle und -wachse sind z.B. Polydimethylsiloxane, Cyclomethylsiloxane, sowie aryl- oder alkyl- oder alkoxy- substituierte Polymethylsiloxane oder Cyclomethylsiloxane. Als weitere Ölkörper kommen beispielsweise Guerbetalkohole auf Basis von Fettalkoholen mit 6 bis 18, vorzugsweise 8 bis 10 Kohlenstoffatomen, Ester von linearen C6-C22-Fettsäuren mit linearen C6- C22-Fettalkoholen, Ester von verzweigten C6-C13-Carbonsäuren mit linearen C₆-C₂₂-Fettalkoholen, Ester von linearen C6-C22-Fettsäuren mit verzweigten C₈-C₁₈-Alkoholen, insbesondere 2-Ethylhexanol oder Isononanol, Ester von verzweigten C₆-C₁₃-Carbonsäuren mit verzweigten Alkoholen, insbesondere 2-Ethylhexanol oder Isononanol, Ester von linearen und/oder verzweigten Fettsäuren mit mehrwertigen Alkoholen (wie z. B. Propylenglycol, Dimerdiol oder Trimertriol) und/oder Guerbetalkoholen, Triglyceride auf Basis C6-C10-Fettsäuren, flüssige Mono-/Di-/Triglyceridmischungen auf Basis von C₆-C₁₈-Fettsäuren, Ester von C₆-C₂₂-Fettalkoholen und/oder Guerbetalkoholen mit aromatischen Carbonsäuren, pflanzliche Öle, verzweigte primäre Alkohole, substituierte Cyclohexane, lineare C₆-C₂₂-Fettalkoholcarbonate, Guerbetcarbonate, Dialkylether, Ringöffnungsprodukte von epoxidierten Fettsäureestern mit Polyolen, Siliconöle und/oder aliphatische bzw. naphthenische Kohlenwasserstoffe in Betracht.

Als Emulgatoren oder Tenside können nichtionische, anionische, kationische oder amphotere Tenside eingesetzt werden. Als nichtionogene Emulgatoren oder Tenside können Verbindungen aus mindestens einer der folgenden Gruppen eingesetzt werden:
Anlagerungsprodukte von 2 bis 100 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe, C_{12/18}-Fettsäuremono- und -diester von Anlagerungsprodukten von 1 bis 100 Mol Ethylenoxid an Glycerin, Glycerinmono- und -diester und Sorbitanmono- und -diester von gesättigten und ungesättigten Fettsäuren mit 6 bis 22 Kohlenstoffatomen und deren Ethylenoxidanlagerungsprodukte, Alkylmono- und -oligoglycoside mit 8 bis 22 Kohlenstoffatomen im Alkylrest und deren Ethylenoxidanlagerungsprodukte, Anlagerungsprodukte von 2 bis 200 Mol Ethylenoxid an Ricinusöl und/oder gehärtetes Ricinusöl, Partialester auf Basis linearer, verzweigter, ungesättigter bzw. gesättigter C₆-C₂₂-Fettsäuren, Ricinolsäure sowie 12-Hydroxystearinsäure und Glycerin, Polyglycerin, Pentaerythrit, Dipentaerythrit, Zuckeralkohole (z.B. Sorbit), Alkylglucoside (z. B. Methylglucosid, Butylglucosid, Laurylglucosid) sowie Polyglucoside (z. B. Cellulose), Mono-, Di- und Trialkylphosphate sowie Mono-, Di- und/oder Tri-PEG-alkylphosphate und deren Salze,
Polysiloxan-Polyether-Copolymere (Dimethicone Copolyole), wie z. B. PEG/PPG-20/6 Dimethicone, PEG/PPG-20/20 Dimethicone, Bis-PEG/PPG-20/20 Dimethicone, PEG-12 oder PEG-14 Dimethicone, PEG/PPG-14/4 oder 4/12 oder 20/20 oder 18/18 oder 17/18 oder 15/15,
Polysiloxan-Polyalkyl-Polyether-Copolymere bzw. entsprechende Derivate, wie z. B. Lauryl oder Cetyl Dimethicone Copolyole, insbesondere Cetyl PEG/PPG-10/1 Dimethicone (ABIL® EM 90 (Evonik Degussa)),
Mischester aus Pentaerythrit, Fettsäuren, Citronensäure und Fettalkohol gemäß DE 11 65 574 und/oder Mischester von Fettsäuren mit 6 bis 22 Kohlenstoffatomen, Methylglucose und Polyolen, wie z. B. Glycerin oder Polyglycerin, Zitronensäureester wie z. B. Glyceryl Stearate Citrate, Glyceryl Oleate Citrate und Dilauryl Citrate.

Anionische Emulgatoren oder Tenside können wasserlöslich machende anionische Gruppen wie z. B. eine Carboxylat-, Sulfat-, Sulfonat- oder Phosphat-Gruppe und einen lipophilen Rest enthalten. Hautverträgliche anionische Tenside sind dem Fachmann in großer Zahl bekannt und im Handel erhältlich. Dabei kann es sich um Alkylsulfate oder Alkylphosphate in Form ihrer Alkali, Ammonium- oder Alkanolammoniumsalze, Alkylethersulfate, Alkylethercarboxylate, Acylsarkosinate sowie Sulfosuccinate und Acylglutamate in Form ihrer Alkali- oder Ammoniumsalze handeln.

Auch kationische Emulgatoren und Tenside können zugesetzt werden. Als solche können insbesondere quaternäre Ammoniumverbindungen, insbesondere solche, versehen mit mindestens einer linearen und/oder verzweigten, gesättigten oder ungesättigten Alkylkette mit 8 bis 22 C-Atomen, eingesetzt werden, so etwa Alkyltrimethylammoniumhalogenide wie z.B. Cetyltrimethylammoniumchlorid oder -bromid oder Behenyltrimethylammoniumchlorid, aber auch Dialkyldimethylammoniumhalogenide wie z. B. Distearyldimethylammoniumchlorid eingesetzt werden.

Weiterhin können Monoalklyamidoquats wie z. B. Palmitamidopropyltrimethylammoniumchlorid oder entsprechende Dialkylamidoquats eingesetzt werden.

Weiterhin können biologisch gut abbaubare quaternäre Esterverbindungen eingesetzt werden, bei denen es sich um quaternierte Fettsäureester auf Basis von Mono-, Di- oder Triethanolamin handeln kann. Weiterhin können Alkylguanidiniumsalze als kationische Emulgatoren beigesetzt sein.

Typische Beispiele für milde, d. h. besonders hautverträgliche Tenside sind Fettalkoholpolyglycolethersulfate, Monoglyceridsulfate, Mono- und/oder Dialkylsulfosuccinate, Fettsäureisethionate, Fettsäuresarcosinate, Fettsäuretauride, Fettsäureglutamate, Ethercarbonsäuren, Alkyloligoglucoside, Fettsäureglucamide, Alkylamidobetaine und/oder Proteinfettsäurekondensate, letztere beispielsweise auf Basis von Weizenproteinen.

Weiterhin ist es möglich, amphotere Tenside wie z. B. Betaine, Amphoacetate oder Amphopropionate einzusetzen, so z.B. Substanzen wie die N-Alkyl-N,N-dimethylammoniumglycinate, beispielsweise das Kokosalkyldimethylammoniumglycinat, N-Acylaminopropyl-N,N-dimethylammoniumglycinate, beispielsweise das Kokosacylaminopropyldimethylammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat.

Von den ampholytischen Tensiden können solche oberflächenaktiven Verbindungen eingesetzt werden, die außer einer C₈/₁₈-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Weitere Beispiele ampholytischer Tenside sind das N-Kokosalkylaminopropionat, das Kokosacylaminoethylaminopropionat und das C₁₂/₁₈-Acylsarcosin.

Geeignete Verdicker sind beispielsweise Polysaccharide, insbesondere Xanthan-Gum, Guar-Guar, Agar-Agar, Alginate und Tylosen, Carboxymethylcellulose und Hydroxyethylcellulose, ferner höhermolekulare Polyethylenglycolmono- und -di-ester von Fettsäuren, Polyacrylate, (z. B. Carbopole TM oder Synthalene TM), Polyacrylamide, Polyvinylalkohol und Polyvinylpyrrolidon, Tenside wie beispielsweise ethoxylierte Fettsäure-glyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid.

Als Verdicker zur Verdickung von Ölphasen kommen alle dem Fachmann bekannten Verdickungsmittel in Frage. Insbesondere sind dabei zu nennen Wachse, wie hydriertes Castorwachs, Bienenwachs oder Microwachs. Weiterhin können auch anorganische Verdickungsmittel eingesetzt werden wie Silica, Alumina oder Schichtsilikate (z. B. Hectorit, Laponit, Saponit). Diese anorganischen Ölphasenverdicker können dabei hydrophob modifiziert sein. Zur Verdickung/Stabilisierung von Wasser-in-Öl-Emulsionen können dabei insbesondere Aerosile, Schichtsilikate und /oder Metallsalze von Fettsäuren, wie z. B. Zinkstearat eingesetzt werden.

Als Viskositätsregler für wässrige Tensidsysteme können z. B. NaCl, niedermolekulare nichtionische Tenside, wie Cocoamide DEA/MEA und Laureth-3, oder polymere, hochmolekulare, assoziative, hochethoxylierte Fettderivate, wie PEG-200 Hydrogenated Glyceryl Palmate enthalten sein.

Als UV-Lichtschutzfilter können beispielsweise organische Substanzen eingesetzt werden, die in der Lage sind, ultraviolette Strahlen zu absorbieren und die aufgenommene Energie in Form längerwelliger Strahlung, z.B. Wärme wieder abzugeben. UVB-Filter können öllöslich oder wasserlöslich sein. Als öllösliche UVB-Lichtschutzfilter sind z. B. zu nennen:
3-Benzylidencampher und dessen Derivate, z.B. 3-(4-Methylbenzyliden)campher (INCI: 4-Methylbenzylidene Campher, Handelsname: Eusolex 6300),
4-Aminobenzoesäurederivate, wie z. B. 4-(Dimethylamino)-benzoesäure-2-ethylhexylester,4-(Dimethylamino)benzoesäure-2-ethylhexylester und 4-(Dimethylamino)benzoesäureamylester Ester der Zimtsäure, wie z. B. 4-Methoxyzimtsäure-2-ethylhexylester (INCI: Octyl Methoxycinnamat, Handelsname: Parsol® MCX), 4-Methoxyzimtsäureisopentylester, 2-Ethylhexyl-2-cyano-3,3-diphenylacrylat (INCI: Octocrylene; Handelsname: Uvinul N-539),
Ester der Salicylsäure, wie z.B. Salicylsäure-2-ethylhexylester, Salicylsäure-4-isopropylbenzylester, Salicylsäurehomomenthylester,
Derivate des Benzophenons, wie z.B. 2-Hydroxy-4-methoxybenzophenon, 2-Hydroxy-4-methoxy-4'-methylbenzophenon, 2,2'-Dihydroxy-4-methoxybenzophenon, 2-(4'-Diethylamino-2'-hydroxybenzoyl)-benzoesäurehexylester (auch: Aminobenzophenon),
Ester der Benzalmalonsäure, wie z. B. 4-Methoxybenzmalonsäuredi-2-ethylhexyester, Triazinderivate, wie z.B. 4,4',4''-(1,3,5-Triazin-2,4,6-triyltriimino)-tris-benzoesäure-tris(2-ethylhexylester) (auch: 2,4,6-Tris-[anilino-(p-carbo-2'-ethyl-1'-hexyloxy)]-1,3,5-triazin, INCI: Octyl Triazone, von der BASF Aktiengesellschaft unter der Warenbezeichnung UVINUL® T 150 vertrieben), 2,4-Bis-{[4-(2-ethyl-hexyloxy)-2-hydroxy]-phenyl}-6-(4-methoxyphenyl)-1,3,5-triazin (INCI: Aniso Triazin, erhältlich unter dem Handelsnamen Tinosorb® S), Dioctylbutylamidotriazon (INCI: Dioctylbutamidotriazone), 2,4-bis-[5-1(dimethylpropyl)benzoxazol-2-yl-(4-phenyl)-imino]-6-(2-ethylhexyl)-imino-1,3,5-triazin mit der (CAS Nr. 288254-16-0, erhältlich bei 3V Sigma unter der Handelsbezeichnung Uvasorb® K2A), 2,4-Bis-{[4-(2-Ethyl-hexyloxy)-2-hydroxy]-phenyl}-6-(4-methoxyphenyl)-1,3,5-triazin (INCI: Bis-Ethylhexyloxylphenol Methoxyphenyl Triazin), erhältlich unter der Handelsbezeichnung Tinosorb® S bei der CIBA-Chemikalien GmbH und 2-[4,6-Bis(2,4-dimethylphenyl)-1,3,5-triazin-2-yl]-5-(octyloxy)phenol (CAS Nr.: 2725-22-6), Propan-1,3-dione, wie z. B. 1-(4-tert.Butylphenyl)-3-(4'-methoxyphenyl)propan-1,3-dion.

Ein weiterer UV-B-Filter ist das 3-(4-(2,2-bis Ethoxycarbonylvinyl)-phenoxy)propenyl)-methoxysiloxan/Dimethylsiloxan-Copolymer, welches beispielsweise unter der Handelsbezeichnung Parsol® SLX erhältlich ist.

Als wasserlösliche UVB-Lichtschutzfilter kommen beispielsweise in Frage:
Salze der 2-Phenylbenzimidazol-5-sulfonsäure, wie ihr Alkali-, Erdalkali-, Ammonium-, Alkylammonium-, Alkanolammonium- und Glucammoniumsalze sowie die Sulfonsäure selbst mit der INCI Bezeichnung Phenylbenzimidazole Sulfonsäure (Handelsname: Eusolex 232), 1,4-di(2-oxo-10-Sulfo-3-bornylidenmethyl)-Benzol (auch: 3,3'-(1,4-Phenylendimethylene)-bis-(7,7-dimethyl-2-oxo-bicyclo-[2.2.1]hept-1-ylmethan Sulfonsäure) und dessen Salze (besonders die entprechenden 10-Sulfato-Verbindungen, insbesondere das entsprechende Natrium-, Kalium- oder Triethanolammonium-Salz), das auch als Benzol-1,4-di(2-oxo-3-bornylidenmethyl-10-sulfonsäure) bezeichnet wird und die INCI-Bezeichnung Terephtalidene Dicampher Sulfonsäure (CAS.-Nr.: 90457-82-2) besitzt, Phenylen-1,4-bis-(2-benzimidazyl)-3,3'-5,5'-tetrasulfonsäure und ihre Salze, die entsprechenden Natrium-, Kalium- oder Triethanolammonium-Salze, z.B. das Phenylen-1,4-bis-(2-benzimidazyl)-3,3'-5,5'-tetrasulfonsäure-bis-natriumsalz mit der INCI-Bezeichnung Bisimidazylate, Handelsname: Neoheliopan® AP (CAS-Nr.: 180898-37-7),
Sulfonsäurederivate von Benzophenon, wie z. B. 2-Hydroxy-4-methoxybenzophenon-5-sulfonsäure und ihre Salze, Sulfonsäurederivate des 3-Benzylidencamphers, wie z. B. 4-(2-Oxo-3-bornylidenmethyl)benzolsulfonsäure und 2-Methyl-5-(2-oxo-3-bornyliden)sulfonsäure und deren Salze.

Als typische UVA-Lichtschutzfilter kommen beispielsweise Derivate des Benzoylmethans in Frage, wie beispielsweise 1-(4'-tert.Butylphenyl)-3-(4'-methoxyphenyl)propan-1,3-dion (4-(tert.-Butyl)-4'-methoxydibenzoylmethan, INCI: Butylmethoxydibenzoylmethan, Handelsname: Parsol 1789) oder 1-Phenyl-3-(4'-isopropylphenyl)propan-1,3-dion.

Die UV-A- und UV-B-Filter können selbstverständlich auch in Mischungen eingesetzt werden.

Als weitere UV-Filtersubstanzen können ferner sogenannte Breitbandfilter, d.h. Filtersubstanzen, die sowohl UV-A-als auch UV-B-Strahlung absorbieren, eingesetzt werden.

Beispiele hierfür sind unter anderen:
2,2'-Methylen-bis-(6-(2H-benzotriazol-2-yl)-4-(1,1,3,3-tetramethylbutyl)-phenol) (Tinosorb® M, CIBA-Chemikalien GmbH), Benzol-1,4-di(2-oxo-3-bornylidenmethyl-10-sulfonsäure) (INCI: Terephtalidene Dicampher Sulfonsäure, Handelsname: Mexoryl® SX), 2-(2H-benzotriazol-2-yl)-4-methyl-6-[2-methyl-3-[1,3,3,3-tetramethyl-1-[(trimethylsilyl)oxy]disiloxanyl]propyl]-phenol (INCI: Drometrizole Trisiloxane, Handelsname: Mexoryl® XL).

Neben den genannten löslichen Stoffen kommen für diesen Zweck auch unlösliche Pigmente, nämlich feindisperse Metalloxide bzw. Salze in Frage, wie beispielsweise Titandioxid, Zinkoxid, Eisenoxid, Aluminiumoxid, Ceroxid, Zirkoniumoxid, Silicate (Talk), Bariumsulfat und Zinkstearat. Die Partikel sollten dabei einen mittleren Durchmesser von weniger als 100 nm, z. B. zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Eine relativ neue Klasse von Lichtschutzfiltern sind mikronisierte organische Pigmente, wie beispielsweise 2,2'-Methylene-bis-{6-(2H-benzotriazole-2-yl)-4-(1, 1, 3, 3-tetramethylbutyl)-phenol} mit einer Partikelgröße von < 200 nm, das z.B. als 50 %ige wässrige Dispersion erhältlich ist.

Des Weiteren sind weitere geeignete UV-Lichtschutzfilter der Übersicht von P. Finkel in SÖFW-Journal 122, 543 (1996) zu entnehmen.

Neben den beiden vorgenannten Gruppen primärer UV-Lichtschutzfilter können auch sekundäre Lichtschutzmittel vom Typ der Antioxidantien eingesetzt werden, die die photochemische Reaktionskette unterbrechen, welche ausgelöst wird, wenn UV-Strahlung in die Haut eindringt. Als Antioxidantien können z.B. Superoxid-Dismutase, Tocopherole (Vitamin E), Dibutylhydroxytoluol und Ascorbinsäure (Vitamin C) eingesetzt werden.

Als Hydrotrope können zur Verbesserung des Fließverhaltens und der Anwendungseigenschaften beispielsweise Ethanol, Isopropylalkohol oder Polyole eingesetzt werden. Polyole, die hier in Betracht kommen, können 2 bis 15 Kohlenstoffatome und mindestens zwei Hydroxylgruppen besitzen. Typische Beispiele sind:
Glycerin Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton, technische Oligoglyceringemische mit einem Eigenkondensationsgrad von 1,5 bis 10 wie etwa technische Diglyceringemische mit einem Diglyceringehalt von 40 bis 50 Gew.-%, Methylolverbindungen, wie insbesondere Trimethylolethan, Trimethylolpropan, Trimethylolbutan, Pentaerythrit und Dipentaerythrit, Niedrigalkylgucoside, insbesondere solche mit 1 bis 4 Kohlenstoffatomen im Alkylrest, wie beispielsweise Methyl- und Butylglucosid, Zuckeralkohole mit 5 bis 12 Kohlenstoffatomen, wie beispielsweise Sorbit oder Mannit, Zucker mit 5 bis 12 Kohlenstoffatomen, wie beispielsweise Glucose oder Saccharose, Aminozucker, wie beispielsweise Glucamin.

Als Feststoffe können beispielsweise Eisenoxidpigmente, Titandioxid oder Zinkoxidpartikel und die zusätzlich unter "UV-Schutzmittel" genannten eingesetzt werden. Weiterhin können auch Partikel eingesetzt werden, die zu speziellen sensorischen Effekten führen, wie etwa Nylon-12, Bornitrid, Polymerpartikel wie etwa Polyacrylat- oder Polymethylacrylatpartikel oder Siliconelastomere. Einsetzbare Füllstoffe umfassen Stärke und Stärkederivate, wie Tapiocastärke, Distärkephosphat, Aluminium- bzw. Natrium-Stärke, Octenylsuccinat sowie Pigmente, die weder hauptsächlich UV-Filternoch färbende Wirkung haben, beispielsweise Aerosile® (CAS-Nr. 7631-86-9).

Als Filmbildner zur z. B. Verbesserung der Wasserfestigkeit können beispielsweise eingesetzt werden: Polyurethane, Dimethicone Copolyol, Polyacrylate oder PVP/VA Copolymer (PVP = Polyvinylpyrrolidon, VA = Vinylacetat). Als fettlösliche Filmbildner können eingesetzt werden: z.B. Polymere auf Basis von Polyvinylpyrrolidon (PVP), Copolymere des Polyvinylpyrrolidons, PVP/Hexadecen-Copolymer oder das PVP/Eicosen-Copolymer.

Als Perlglanzadditive können z.B. Glycoldistearate oder PEG-3 Distearat eingesetzt werden.

Als Deodorantwirkstoffe kommen z. B. Geruchsüberdecker wie die gängigen Parfümbestandteile, Geruchsabsorber, beispielsweise die in der Patentoffenlegungsschrift DE 40 09 347 beschriebenen Schichtsilikate, von diesen insbesondere Montmorillonit, Kaolinit, Ilit, Beidelit, Nontronit, Saponit, Ilectorit, Bentonit, Smectit, ferner beispielsweise Zinksalze der Ricinolsäure in Betracht. Keimhemmende Mittel sind ebenfalls geeignet, eingearbeitet zu werden. keimhemmende Substanzen sind zum Beispiel 2,4,4'-Trichlor-2'-hydroxydiphenylether (Irgasan), 1,6-Di-(4-chlorphenylbiguanido)-hexan (Chlorhexidin), 3,4,4'-Trichlorcarbanilid, quaternäre Ammoniumverbindungen, Nelkenöl, Minzöl, Thymianöl, Triethylcitrat, Farnesol (3,7,11-Trimethyl-2,6,10-dodecatrien-1-ol), Ethylhexyl glycerylether, Polyglyceryl-3 caprylat (TEGO® Cosmo P813, Evonik Degussa), sowie die in den Patentoffenlegungsschriften DE 198 55 934, DE 37 40 186, DE 39 38 140, DE 42 04 321, DE 42 29 707, DE 42 29 737, DE 42 38 081, DE 43 09 372, DE 43 24 219 und EP 0 666 732 beschriebenen wirksamen Agenzien.

Als Antitranspirantwirkstoffe können Adstringentien eingesetzt werden, beispielsweise basische Aluminiumchloride wie Aluminiumchlorhydrat ("ACH") und Aluminium-Zirkonium-Glycine-Salze ("ZAG").

Als Insekten-Repellentien können beispielsweise N,N-Diethyl-m-toluamid, 1,2-Pentandiol oder Insect Repellent 3535 eingesetzt werden.

Als Selbstbräuner können z. B. Dihydroxyaceton und Erythrulose eingesetzt werden.

Als Konservierungsstoffe können beispielsweise Mischungen einzelner oder mehrerer Alkylparabenester mit Phenoxyethanol eingesetzt werden. Bei den Alkylparabenestern kann es sich um Methlyparaben, Ethylparaben, Propylparaben und/oder Butylparaben handeln. Anstelle von Phenoxyethanol können auch andere Alkohole eingesetzt werden, wie beispielsweise Benzylalkohol oder Ethanol. Darüber hinaus können auch andere übliche Konservierungsmittel wie etwa Sorbin- oder Benzoesäure, Salicylsäure, 2-Bromo-2-Nitropropan-1,3-Diol, Chloracetamid, Diazolidinyl Harnstoff, DMDM Hydantoin, Iodopropynyl Butylcarbamat, Natrium Hydroxymethylglycinate, Methylisothiazolin, Chlormethyl-isothiazolin, Ethylhexylglycerin oder Caprylyl Glycol eingesetzt werden.

Als Konditioniermittel können z.B. organische quaternäre Verbindungen wie Cetrimoniumchlorid, Dicetyldimoniumchlorid, Behentrimoniumchlorid, Distearyldimoniumchlorid, Behentrimoniummethosulfat, Distearoylethyldimoniumchlorid, Palmitamidopropyltrimoniumchlorid, Guar Hydroxypropyltrimoniumchlorid, Hydroxypropylguar Hydroxypropyltrimoniumchlorid, oder Quaternium-80 oder auch Aminderivate wie z.B.

Aminopropyldimethicone oder Stearamidopropyldimethylamine verwendet werden.

Als Parfüme können natürliche oder synthetische Riechstoffe oder Gemische daraus eingesetzt werden. Natürliche Riechstoffe sind Extrakte von Blüten (Lilie, Lavendel, Rosen, Jasmin, Neroli, Ylang-Ylang), Stengeln und Blättern (Geranium, Patchouli, Petitgrain), Früchten (Anis, Koriander, Kümmel, Wacholder), Fruchtschalen (Bergamotte, Zitrone, Orange), Wurzeln, (Macis, Angelica, Sellerie, Kardamon, Costus, Iris, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethyl-phenylglycinat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroycitronellal, Lilial und Bourgeonal, zu den Ketonen z.B. die Jonone, α-Isomethylionon und Methyl-cedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Es können Mischungen verschiedener Riechstoffe eingesetzt werden, die gemeinsam eine ansprechende Duftnote erzeugen. Auch ätherische Öle geringer Flüchtigkeit, die meist als Aromakomponenten eingesetzt werden, eignen sich als Parfüme, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labolanumöl und Lavandinöl. Es können Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romillat, Irotyl und Floramat allein oder in Mischungen, eingesetzt werden.

Als Farbstoffe können die für kosmetische Zwecke geeigneten und zugelassenen Substanzen eingesetzt werden, wie sie beispielsweise in der Publikation "Kosmetische Färbemittel" der Farbstoffkommission der Deutschen Forschungsgemeinschaft, Verlag Chemie, Weinheim, 1984, S. 81 bis 106 zusammengestellt sind. Diese Farbstoffe werden üblicherweise in Konzentrationen von 0,001 bis 0,1 Gew.-%, bezogen auf die gesamte Mischung, eingesetzt.

Unter biogenen Wirkstoffen sind beispielsweise Tocopherol, Tocopherolacetat, Tocopherolpalmitat, Ascorbinsäure, Desoxyribonucleinsäure, Coenzym Q10, Retinol, Bisabolol, Allantoin, Phytantriol, Panthenol, AHA-Säuren, Aminosäuren, Hyaluronsäure, alpha-Hydroxysäuren, Polyglutaminsäure, Creatin (und Creatinderivate), Guanidin (und Guanidinderivate), Ceramide, Phytosphingosin (und Phytosphingosinderivate), Sphingosin (und Sphingosinderivate), Pseudoceramide, essentielle Öle, Peptide, Proteinhydrolysate, Pflanzenextrakte, Sphingolipide und Vitaminkomplexe zu verstehen.

Als Pflegeadditive können z. B. ethoxylierte Glycerin-Fettsäureester, wie beispielweise PEG-7 Glycerin Cocoate, oder kationische Polymere, wie beispielsweise Polyquaternium-7 oder Polyglycerinester enthalten sein.

Als Überfettungsmittel können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen.

Als Lösungsmittel können z. B. aliphatische Alkohole wie Ethanol, Propanol oder 1,3-Propandiol, cyclische Carbonate wie Ethylencarbonat, Propylencarbonat, Glycerincarbonat, Ester von Mono- oder Polycarbonsäuren wie Ethylacetat, Ethyllactat, Dimethyladipat und Diethyladipat, Propylenglycol, Dipropylenglycol, Glycerin, Glycerincarbonat oder Wasser eingesetzt werden.

Erfindungsgemäße Verwendung eignet sich insbesondere für die Herstellung von Sonnenschutzpräparaten.

Ein weiterer Gegenstand der Erfindung ist daher die Verwendung von Isononylbenzoat, bevorzugt von einer oder mehreren Substanzen der Formel (I), mit der Maßgabe, dass die durch Verseifung der verwendeten Substanz(en) erhaltenen Nonylalkohole weniger als 80 Mol %, bevorzugt weniger als 40 Mol % und besonders bevorzugt weniger als 10 Mol % 3,5,5-Trimethylhexanol enthalten, in kosmetischen, dermatologischen oder pharmazeutischen Formulierungen als Lösungsvermittler oder Lösungsmittel von mindestens einer UV-Lichtschutzfiltersubstanz.

Daher ist auch eine bevorzugt eingesetzte, zusätzliche Komponente die Gruppe der Substanzen der UV-Lichtschutzfilter. Hier werden bevorzugt die lipophilen, hydrophoben UV-Lichtschutzfiltersubstanzen, insbesondere Triazinderivate, eingesetzt.

Besonders bevorzugt werden hier als UV-B-Filter die UV-Lichtschutzfiltersubstanzen 2-Cyano-3-phenyl-zimtsäure-2-ethylhexylester, 2,4-Bis-{[4-(2-ethyl-hexyloxy)-2-hydroxy]-phenyl}-6-(4-methoxyphenyl)-1,3,5-triazin, Dioctylbutylamidotriazon, 2-Hydroxy-4-methoxybenzophenon, 2-Hydroxy-4-methoxy-4'-methylbenzophenon, 2,2'-Dihydroxy-4-methoxybenzophenon, 4-Methoxybenzmalonsäuredi-2-ethylhexylester, 2,4,6-Tris-[anilino-(p-carbo-2'-ethyl-1'-hexyloxy)]-1,3,5-triazin, 2,4-bis-[5-1(dimethylpropyl)benzoxazol-2-yl-(4-phenyl)-imino]-6-(2-ethylhexyl)-imino-1,3,5-triazin, 2,4-Bis-{[4-(2-Ethyl-hexyloxy)-2-hydroxy]-phenyl}-6-(4-methoxyphenyl)-1,3,5-triazin und 2-[4,6-Bis(2,4-dimethylphenyl)-1,3,5-triazin-2-yl]-5-(octyloxy)phenol eingesetzt.

Als UV-A-Filter werden vorzugsweise 1-(4'-tert.Butylphenyl)-3-(4'-methoxyphenyl)propan-1,3-dion, 1-Phenyl-3-(4'-isopropylphenyl)-propan-1,3-dion eingesetzt. Besonders bevorzugte UV-A-Filter sind 4-(tert.-Butyl)-4'-methoxydibenzoylmethan (CAS-Nr. 70356-09-1), welches von Givaudan unter der Marke Parsol® 1789 und von Merck unter der Handelsbezeichnung Eusolex® 9020 verkauft wird, und Hydroxybenzophenone gemäß DE 102004027475, besonders bevorzugt der 2-(4'-Diethylamino-2'-hydoxybenzoyl)-benzoesäurehexylester (auch: Aminobenzophenon), welcher unter Namen Uvinul A Plus bei der Fa. BASF erhältlich ist, eingesetzt.

Weiterhin bevorzugte UV-Filtersubstanzen sind ferner sogenannte Breitbandfilter, d.h. Filtersubstanzen, die sowohl UV-A- als auch UV-B-Strahlung absorbieren. Innerhalb dieser Gruppe werden bevorzugt 2,2'-Methylen-bis-(6-(2H-benzotriazol-2-yl)-4-(1,1,3,3-tetramethylbutyl)-phenol, welches unter der Handelsbezeichnung Tinosorb® M bei der CIBA-Chemikalien GmbH erhältlich ist und 2-(2H-benzotriazol-2-yl)-4-methyl-6-[2-methyl-3-[1,3,3,3-tetramethyl-1-[(trimethylsilyl)oxy]disiloxanyl]propyl]-phenol (CAS-Nr.: 155633-54-8) mit der INCI-Bezeichnung Drometrizole Trisiloxane, eingesetzt.

Die Einsatzmenge der UV-Lichtschutzfilter beträgt vorzugsweise 0,01-15 %, bevorzugt 0,05-10 %, besonders bevorzugt 0,1-5 % bezogen auf die Formulierung.

Bevorzugt ist der Einsatz einer Kombination aus mehreren verschiedenen UV-Filtern.

Eine weitere, bevorzugt eingesetzte, zusätzliche Komponente ist die Gruppe der Filmbildner, um die Wasserfestigkeit der Zusammensetzungen zu verbessern und somit auch die UV-Schutzleistung zu erhöhen. Bevorzugt eingesetzte Filmbildner sind Polyurethane, Dimethicone Copolyol, Polyacrylate, PVP/VA Copolymer (PVP = Polyvinylpyrrolidon, VA = Vinylacetat), Polyvinylpyrrolidon (PVP), Copolymere des Polyvinylpyrrolidons, PVP/Hexadecen-Copolymer oder das PVP/Eicosen-Copolymer.

Eine weitere, bevorzugt eingesetzte, zusätzliche Komponente ist die Gruppe der Deodorant- und Antitranspirantwirkstoffe. Aus dieser Gruppe werden bevorzugt Adstringentien, besonders bevorzugt basische Aluminiumchloride wie Aluminiumchlorhydrat ("ACH") oder Aluminium-Zirkonium-Glycine-Salze ("ZAG") eingesetzt.

Gegenstand der Erfindung sind außerdem kosmetische, dermatologische oder pharmazeutische Formulierungen, enthaltend eine Substanz der allgemeinen Formel (I) wobei R₁ ein unverzweigter oder verzweigter C₉-Alkylrest ist.

Erfindungsgemäße Formulierungen können beispielsweise Verwendung als ein Hautpflege-, Gesichtspflege-, Kopfpflege-, Körperpflege, Intimpflege-, Fußpflege-, Haarpflege-, Nagelpflege, Zahnpflege- oder Mundpflegeprodukt finden.

Erfindungsgemäße Formulierungen können beispielsweise Verwendung in Form einer Emulsion, einer Suspension, einer Lösung, einer Creme, einer Salbe, einer Paste, eines Gels, eines Öls, eines Puders, eines Aerosols, eines Stiftes, eines Sprays, eines Reinigungsproduktes, eines Schmink- oder Sonnenschutzpräparates oder eines Gesichtswassers finden.

Vorzugsweise enthalten die kosmetischen, dermatologischen oder pharmazeutischen Formulierungen 0,1 bis 60 Gewichtsprozent, bevorzugt 1 bis 25 Gewichtsprozent und besonders bevorzugt 3 bis 15 Gewichtsprozent mindestens einer Substanz der allgemeinen Formel (I).

Besonders bevorzugt enthalten die kosmetischen, dermatologischen oder pharmazeutischen Formulierungen eine oder mehrere Substanzen der Formel (I), mit der Maßgabe, dass die durch Verseifung der verwendeten Substanz(en) erhaltenen Nonylalkohole weniger als 80 Mol %, vorzugsweise weniger als 40 Mol % und besonders bevorzugt weniger als 10 Mol % 3,5,5-Trimethylhexanol enthalten.

Besonders bevorzugte kosmetische, dermatologische oder pharmazeutische Formulierungen enthalten:
(a) 0,1 - 60 Gewichtsprozent mindestens einer Substanz der allgemeinen Formel (I), bevorzugt von einer oder mehreren Substanzen der Formel (I), mit der Maßgabe, dass die durch Verseifung der verwendeten Substanz(en) erhaltenen Nonylalkohole weniger als 10 Mol % 3,5,5-Trimethylhexanol enthalten,
(b) 0,1 - 20 Gewichtsprozent Tenside und/oder Emulgatoren und/oder Coemulgatoren,
(c) 0,1 - 40 Gewichtsprozent weiterer Ölkörper und
(d) 0 - 98 Gewichtsprozent Wasser,
wobei die Gewichtsprozent der Komponenten (a), (b), (c) und
(d) sich auf 100 Gewichtsprozent addieren.

In den nachfolgend aufgeführten Beispielen wird die vorliegende Erfindung beispielhaft beschrieben, ohne dass die Erfindung, deren Anwendungsbreite sich aus der gesamten Beschreibung und den Ansprüchen ergibt, auf die in den Beispielen genannten Ausführungsformen beschränkt sein soll.

### Beispiele:

### Beispiel 1: Lösungsvermögen

Zum Test des Lösungsvermögens von kristallinen UV-Lichtschutzfiltern in Isononylbenzoat (Vestinol ® INB, Evonik Oxeno GmbH) wurden stellvertretend vier repräsentative UV-A- bzw. UV-B-Lichtschutzfilter ausgewählt. Hierbei handelt es sich um Benzophenon-3 (2-Hydroxy-4-methoxy-benzophenon, BP-3), Butyl Methoxydibenzoylmethane (BMDM), Octyl Triazon (2,4,6-Trianilino-p-(carbo-2'ethylhexyl-1'-oxi)-1,3,5-triazine, EHT) und Bemotrizinol (bis-ethylhexyloxyphenol methoxyphenyl triazine, BEMT).

Das Lösungsvermögen von herkömmlichen Esterölen für diese Verbindungen ist in den meisten Fällen nicht befriedigend. Eine Verbindung mit gutem Lösungsvermögen für UV-Lichtschutzfiltersubstanzen ist das schon erwähnte Tegosoft^{®} TN, welches deshalb als Inhaltsstoff für Sonnenschutzformulierungen auch weithin etabliert ist.

Zur Bestimmung des Lösungsvermögens für diese drei UV-Lichtschutzfiltern wird jeweils eine bestimmte Menge (50g) einer der erfindungsgemäßen Verbindungen vorgelegt und auf 22°C temperiert. Nun wird 1 Gewichtsprozent eines UV-Lichtschutzfilters zugegeben und gerührt, bis sich diese Menge vollständig und homogen gelöst hat. Dieser Vorgang wird wiederholt, bis die maximal lösbare Menge des UV-Lichtschutzfilters überschritten wurde. Zum vollständigen Auflösen ist bei höheren Konzentrationen oftmals eine längere Rührzeit von mehreren Stunden erforderlich.

Ist auf diese Weise die Maximalkonzentration grob bestimmt worden, wird zur Feinbestimmung der Konzentrationsbereich um diese Maximalkonzentration herum mit kleineren Einwaagemengen des UV-Lichtschutzfilters wiederholt.

Als Referenz wurde die Verbindung Tegosoft^{®} TN (C12-15 Alkyl Benzoate) verwendet.

| | *Uv-Filter* | | | |
|---|---|---|---|---|
| *Produkt* | *BP-3* | *BMDM* | *EHT* | *BEMT* |
| Tegosoft® TN (C12-15 Alkyl Benzoat) | 15% | 13% | 4% | 9% |
| Isononyl Benzoat | 21% | 14% | 6% | 12% |

Wie aus den obigen Werten ersichtlich, ist das Lösungsvermögen der erfindungsgemäßen Verbindungen in vielen Fällen deutlich besser als das Lösungsvermögen von Tegosoft® TN.

### Beispiel 2: Kosmetische Formulierungen:

Die beschriebenen kosmetischen Emulsionen sollen dazu dienen, die Verwendbarkeit von Isononylbenzoat in kosmetischen Emulsionen beispielhaft zu illustrieren.
Dabei kann Isononylbenzoat sowohl in Öl-in-Wasser als auch in Wasser-in-Öl Emulsionen eingesetzt werden. Weiterhin können kosmetische Emulsionen, sowohl flüssiger als auch pastöser Konsistenz, mit Isononylbenzoat sowohl in Heiß-Prozessen als auch in Kaltprozessen hergestellt werden. Herstellung und Homogensierschritte erfolgen nach üblichen Methoden. Als Isononylbenzoat wurde immer Vestinol ® INB, Evonik Oxeno GmbH eingesetzt.

**Formulierungsbeispiel 1: O/W Pflegecreme (Heißherstellung)**

| | %w/w |
|---|---|
| AXOL^{®} C 62 | 1, 5 |
| TEGIN^{®} M | 2,0 |
| TEGO^{®} Alkanol 1618 | 3,0 |
| Isononyl Benzoat | 4,7 |
| TEGOSOFT^{®} CR | 2, 0 |
| TEGOSOFT^{®} TIS | 1,0 |
| TEGOSOFT^{®} MM | 0,5 |
| Cyclomethicone | 4,0 |
| Macadamia Ternifolia Seed Oil | 2,0 |
| Tocopheryl Acetate | 0,5 |
| Glycerin | 4,0 |
| Panthenol | 0,5 |
| Allantoin | 0,2 |
| Wasser | 65,5 |
| TEGO^{®} Carbomer 134 | 0,3 |
| Bisabolol | 0,5 |
| NaOH (10%ige wässrige Lösung) | 0,8 |
| Ethanol | 7,0 |
| Parfüm, Konservierungsmittel | q.s. |

**Formulierungsbeispiel 2: O/W Body Lotion (Kaltherstellung)**

| | %w/w |
|---|---|
| TEGO^{®} Care LTP | 2, 0 |
| Isononyl Benzoat | 7,3 |
| TEGOSOFT^{®} CT | 6,5 |
| TEGO^{®} Carbomer 141 | 0,1 |
| TEGO^{®} 341 ER | 0,1 |
| Glycerin | 3,0 |
| Wasser | 80,4 |
| NaOH (10%ige wässrige Lösung) | 0,6 |
| Parfüm, Konservierungsmittel | q.s. |

**Formulierungsbeispiel 3: O/W Tagescreme (Heißherstellung)**

| | %w/w |
|---|---|
| TEGO^{®}Care 450 | 3,0 |
| TEGIN^{®} M | 2,0 |
| TEGO^{®}Alkanol 18 | 1,0 |
| Isononyl Benzoat | 4,5 |
| TEGOSOFT^{®}Liquid | 9,5 |
| TEGOSOFT^{®} P | 7,5 |
| Ethylhexyl Methoxycinnamate | 2,0 |
| Tocopheryl Acetate | 0,5 |
| Glycerin | 3,0 |
| Wasser | 66,2 |
| TEGO^{®} Carbomer 134 | 0,2 |
| NaOH (10%ige wässrige Lösung) | 0,6 |
| Parfüm, Konservierungsmittel | q.s. |

**Formulierungsbeispiel 4: W/O Body Lotion**

| | %w/w |
|---|---|
| ISOLAN^{®} GPS | 3,0 % |
| Hydrogenated Castor Wax | 0,25 % |
| Microcrystalline Wax | 0.25 % |
| Isononyl Benzoat | 10,7 % |
| TEGOSOFT^{®} DEC | 10.0 % |
| Tocopheryl Acetate | 0,6 % |
| TEGO^{®} Cosmo C 100 | 0,5 % |
| Panthenol | 0,5 % |
| Glycerin | 3,0 % |
| Wasser | 70,2 % |
| MgSO₄ * 7 H₂O | 1,0 % |
| Parfüm, Konservierungsmittel | q.s. |

**Formulierungsbeispiel 5: W/O-Sonnenschutzformulierungen**

| | Nr. 5.1 | Nr. 5.2 | Nr. 5.3 |
|---|---|---|---|
| | %w/w | %w/w | %w/w |
| ISOLAN® GPS | 3,0 | 3,0 | 3,0 |
| Hydrogenated Castor Wax | 0,2 | 0,2 | 0,2 |
| Microcrystalline Wax | 0,2 | 0,2 | 0,2 |
| Isononyl Benzoat | 13,5 | 13,5 | 7,6 |
| Octocrylene | ---- | ---- | 8,0 |
| Ethylhexyl Methoxycinnamate | 4,0 | ---- | ---- |
| Ethylhexyl Salicylate | ---- | ---- | 5,0 |
| Butyl Methoxydibenzoylmethane | 3,0 | ---- | 2,0 |
| TEGO^{®} Sun TDEC 45 | 11,0 | 11,0 | ---- |
| TEGO^{®} Sun Z 500 | ---- | 3,0 | ---- |
| Isostearic Acid | ---- | 1,0 | ---- |
| Wasser | 61,5 | 64,6 | 70,5 |
| Glycerin | 2,0 | 2,0 | 2,0 |
| MgSO4*7H2O | 1,5 | 1,5 | 1,5 |
| Parfüm, Konservierungsmittel | q.s. | q.s. | q.s. |
| SPF (Optometrics) | 38 | 11 | 20 |

**Formulierungsbeispiel 6: O/W Sonnenschutzlotion**

| | Nr. 6.1 | Nr. 6.2 | Nr. 6.3 | Nr. 6.4 |
|---|---|---|---|---|
| | %w/w | %w/w | %w/w | %w/w |
| Axol^{®}C 62 | 2,0 | 2,5 | -- | -- |
| TEGO^{®}Alkanol 1618 | 1, 0 | 1, 0 | -- | 1,0 |
| TEGO Care 450 | -- | -- | 3,0 | 3,0 |
| TEGIN^{®}M | -- | -- | -- | 2,0 |
| Isononyl Benzoat | 9,0 | 5,5 | 8,5 | 16,5 |
| Jojoba Oil | -- | -- | 2,5 | 2,0 |
| Tocopheryl Acetate | 0,5 | 0,5 | -- | -- |
| TEGO^{®}Sun TDEC 45 | 5,0 | -- | -- | -- |
| TEGO^{®}Sun T 805 | -- | 3,0 | -- | -- |
| TEGO^{®}Sun Z 500 | -- | -- | -- | 5,0 |
| Ethylhexyl Methoxycinnamate | 7,0 | 7,0 | 7,5 | -- |
| Ethylhexyl Salicylate | -- | -- | 5,0 | -- |
| Butyl Methoxydibenzoyl-methane | 3,0 | 2,0 | -- | -- |
| Benzophenone-3 | -- | -- | 5,0 | -- |
| Menthyl Antranilate | -- | -- | 5,0 | -- |
| bis-ethylhexyloxyphenol methoxyphenyl triazine | -- | -- | -- | 3,0 |
| Xanthan Gum | -- | 0,3 | -- | -- |
| Glycerin | 2,0 | 2,0 | -- | -- |
| Sodium Hexamethaphosphate (Graham's Salt) | -- | -- | -- | 0,2 |
| Wasser | 68,9 | 74, 6 | 61,45 | 63, 9 |
| Tego^{®} Carbomer 141 | 0,2 | 0,2 | 0,25 | -- |
| TEGOSOFT^{®} TN | 0,8 | 0,8 | 1,0 | -- |
| NaOH (10%ige wäßrige Lösung) | 0,6 | 0,6 | 0,8 | -- |
| ZnSO₄ * 7 H₂O | -- | -- | -- | 0,4 |
| Parfüm, Konservierungsmittel | q.s. | q.s. | q.s. | q.s. |
| | | | | |
| SPF (Optometrics) | 48 | 35 | 30 | 15 |

**Formulierungsbeispiel 7: O/W-Sonnenschutzcreme**

| (Kaltherstellung) | |
|---|---|
| | %w/w |
| ABIL^{®} Care 85 | 2, 0 |
| Isononyl Benzoat | 5,5 |
| TEGOSOFT^{®} DC | 3,0 |
| Tocopheryl Acetate | 0,5 |
| Isoamyl Methoxycinnamate | 4,0 |
| Ethylhexyl Methoxycinnamate | 4,0 |
| Octocrylene | 4,0 |
| Butyl Methoxydibenzoylmethane | 2,0 |
| TEGO^{®} SMO 80V | 1,0 |
| Glycerin | 2,0 |
| Wasser | 71,1 |
| TEGO^{®} Carbomer 141 | 0, 05 |
| TEGO^{®} Carbomer 341ER | 0, 05 |
| TEGOSOFT^{®} DC | 0,4 |
| NaOH (10%ige wässrige Lösung) | 0,4 |
| Parfüm, Konservierungsmittel | q.s. |
| SPF (Optometrics) | 35 |

**Formulierungsbeispiel 8: O/W_onnenschutzspray**

| | %w/w |
|---|---|
| Isononyl Benzoat | 5,6 |
| TEGOSOFT^{®} DC | 2,5 |
| Tocopheryl Acetate | 0,5 |
| Ethylhexyl Methoxycinnamate | 5,0 |
| Octocrylene | 3,0 |
| Butyl Methoxydibenzoylmethane | 2,0 |
| TEGO^{®} Care CG 90 | 1,0 |
| Glycerin | 2,0 |
| Wasser | 77,5 |
| TEGO^{®} Carbomer 141 | 0, 05 |
| TEGO^{®} Carbomer 341ER | 0, 05 |
| TEGOSOFT^{®} DC | 0,4 |
| NaOH (10%ige wässrige Lösung) | 0,4 |
| Parfüm, Konservierungsmittel | q.s. |
| SPF (Optometrics) | 13 |

## Patentansprüche

1. Verwendung mindestens einer Substanz der allgemeinen Formel (I) 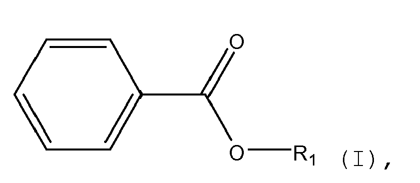 wobei R₁ ein unverzweigter oder verzweigter C₉-Alkylrest ist,
zur Herstellung einer kosmetischen, dermatologischen oder pharmazeutischen Formulierung.

2. Verwendung gemäß Anspruch 1, **dadurch gekennzeichnet, dass** mindestens eine Substanz der Formel (I) verwendet wird, mit der Maßgabe, dass die durch Verseifung der verwendeten Substanz oder Substanzen erhaltenen Nonylalkohole weniger als 80 Mol-% 3,5,5-Trimethylhexanol enthalten.

3. Verwendung gemäß Anspruch 1 oder 2, **dadurch gekennzeichnet, dass** mindestens eine Substanz der Formel (I) als Lösungsvermittler oder Lösungsmittel für mindestens eine UV-Lichtschutzfiltersubstanz eingesetzt wird.

4. Verwendung gemäß Anspruch 3, **dadurch gekennzeichnet, dass** mindestens eine der UV-Lichtschutzfiltersubstanzen ein Triazinderivat ist.

5. Verwendung gemäß Anspruch 3 oder 4, **dadurch gekennzeichnet, dass** mindestens eine der UV-Lichtschutzfiltersubstanzen ausgewählt ist aus der Gruppe der Verbindungen, enthaltend
2-Cyano-3-phenyl-zimtsäure-2-ethylhexylester,
2,4-Bis-{[4-(2-ethyl-hexyloxy)-2-hydroxy]-phenyl}-6-(4-methoxyphenyl)-1,3,5-triazin,
Dioctylbutylamidotriazon,
2-Hydroxy-4-methoxybenzophenon,
2-Hydroxy-4-methoxy-4'-methylbenzophenon,
2,2'-Dihydroxy-4-methoxybenzophenon,
4-Methoxybenzmalonsäuredi-2-ethylhexylester,
2,4,6-Tris-[anilino-(p-carbo-2'-ethyl-1'-hexyloxy)]-1,3,5-triazin,
2,4-bis-[5-1(dimethylpropyl)benzoxazol-2-yl-(4-phenyl)-imino]-6-(2-ethylhexyl)-imino-1,3,5-triazin,
2,4-Bis-{[4-(2-Ethyl-hexyloxy)-2-hydroxy]-phenyl}-6-(4-methoxyphenyl)-1,3,5-triazin und
2-[4,6-Bis(2,4-dimethylphenyl)-1,3,5-triazin-2-yl]-5-(octyloxy)phenol.

6. Kosmetische, dermatologische oder pharmazeutische Formulierung enthaltend mindestens eine Substanz der allgemeinen Formel (I) 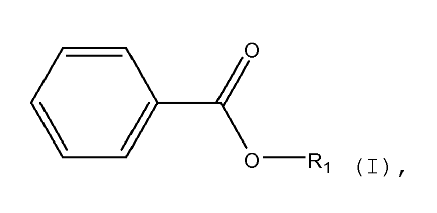 wobei R₁ ein unverzweigter oder verzweigter C₉-Alkylrest ist.

7. Kosmetische, dermatologische oder pharmazeutische Formulierung gemäß Anspruch 6, **dadurch gekennzeichnet, dass** sie
(a) 0,1 - 60 Gewichtsprozent mindestens einer Substanz der allgemeinen Formel (I),
(b) 0,1 - 20 Gewichtsprozent Tenside und/oder Emulgatoren und/oder Coemulgatoren,
(c) 0,1 - 40 Gewichtsprozent weiterer Ölkörper
(d) 0 - 98 Gewichtsprozent Wasser
enthält,
wobei die Gewichtsprozente der Komponenten (a), (b),
(c) und (d) sich auf 100 Gewichtsprozent addieren.

8. Formulierung gemäß Anspruch 6 oder 7, **dadurch gekennzeichnet, dass** mindestens eine Substanz der Formel (I) enthalten ist, mit der Maßgabe, dass die durch Verseifung der enthaltenen Substanz oder Substanzen der Formel (I) erhaltenen Nonylalkohole weniger als 80 Mol-% 3,5,5-Trimethylhexanol enthalten.
